# EUROPÄISCHE PATENTSCHRIFT

(11) **EP 1 484 341 B1**
(45) Veröffentlichungstag und Bekanntmachung des Hinweises auf die Patenterteilung: **08.11.2006**
(21) Anmeldenummer: 04012051.1
(22) Anmeldetag: 21.05.2004
(51) Int. Cl.: C08B 31/04, C08B 37/00, C07H 13/02

(54) **Herstellung von Saccharidestern**
Preparation of saccharide esters
Préparation d'esters de saccharide

(30) Priorität: 04.06.2003 DE 10325200
(43) Veröffentlichungstag der Anmeldung: 08.12.2004
(73) Patentinhaber: Clariant Produkte (Deutschland) GmbH, 65929 Frankfurt am Main (DE)
(72) Erfinder: Scherl, Franz-Xaver Dr., 84508 Burgkirchen (DE); Klug, Peter, Dr., 63762 Grossostheim (DE); Potyka, Claudia, 84556 Kastl (DE)

(56) Entgegenhaltungen:
- DE-A- 4 423 681
- US-A- 5 470 969
- US-A- 5 840 883

## Beschreibung

Die vorliegende Erfindung betrifft ein Verfahren zur Herstellung von Saccharidestern in aprotischen Lösungsmitteln ohne Zusatz einer Aminbase.

Verbraucherwünsche und Rheologie kosmetischer Produkte sind eng miteinander verknüpft. So wird z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Deodorantien, Sonnenschutzfilter) und auch die Lagerstabilität der Formulierung steht in enger Abhängigkeit zu den rheologischen Eigenschaften des Produktes. Im kosmetischen Bereich kommt daher den Verdickungsmitteln und Gelbildnern eine tragende Rolle zu.

Eine Reihe von Patentschriften beschreiben den Einsatz von Polyolestern als Verdickungsmittel. In DE 37 26 015 werden Umsetzungsprodukte von Polyalkoholen mit Fettsäuren, beispielsweise Pentaerythritolfettsäureester und deren verdickende Wirkung, beschrieben. Hochthixotrope Mittel zur Verdickung von Ölphasen können durch Verwendung von Dextrinestern hergestellt werden, deren Herstellung in US 5,840,883 beschrieben wird. Die Umsetzung von Dextrin mit einem Fettsäurehalogenid oder -anhydrid erfolgt in Dimethylformamid oder Formamid, Acetamidverbindungen, Ketonverbindungen, Aromaten, beispielsweise Benzol, Toluol, Xylol, Dioxan als Lösungs- oder Dispergiermittel in Gegenwart eines tertiären Amins, beispielsweise Pyridin, Triethylamin oder Picolin. Tertiäre Amine sind toxisch, beeinträchtigen den Geruch der Mittel und erfordern einen hohen Aufwand bezüglich Reinigung der Dextrinester und Aufarbeitung der herstellbedingten Abfallstoffe. In WO 00/61079 und US 2002/0072506 wird die Herstellung von Cellobioseestern durch Umsetzung von Cellobiose und Säureanhydrid in Substanz beschrieben, wobei jedoch unbefriedigende Ausbeuten erzielt werden.

In US 5,470, 969 wird die Monoacylierung von Saccharose in 6-Position durch Umsetzung von Saccharose mit einem Carbonsäureanhydrid in einer Reaktionsmischung enthaltend ein polar aprotisches Lösungsmittel und 1,3-Diacyloxy-1,1,3,3-tetra(hydrocarbyl)distannoxan beschrieben.

DE 44 23 681 betrifft ein Verfahren zur Herstellung von Acetaten von Stärke oder ihren Hauptkomponenten Amylose und Amylopektin und ermöglicht es, diese Acetate mit einem beliebigen Durchschnitts-Substitutionsgrad (DS) von > 0,5 unter Verwendung äquimolarer Mengen von Acetanhydrid als Acylierungsmittel reproduzierbar zu synthetisieren.

Es bestand daher die Aufgabe, ein neues Verfahren zur Herstellung von Saccharidestern zu finden, welches hohe Produktausbeuten und gute Qualitäten liefert.

Überraschenderweise können Saccharidester in sehr guten Ausbeuten erhalten werden, wenn Saccharide mit aktivierten Carbonsäuren ausgewählt aus Carbonsäurechloriden in Abwesenheit einer Aminbase wie z.B. Pyridin, umgesetzt werden, und die Veresterung der Saccharide in einem dipolar aprotischen Lösungsmittel ausgewählt aus der Gruppe der Lactame stattfindet.

Gegenstand der Erfindung ist ein Verfahren zur Herstellung von Saccharidestern durch Umsetzung von Sacchariden mit aktivierten Carbonsäuren ausgewählt aus Carbonsäurechloriden in einem dipolar aprotischen Lösungsmittel ausgewählt aus der Gruppe der Lactame und in Abwesenheit einer basischen Aminverbindung.

Neben den hohen Ausbeuten, die nach dem erfindungsgemäßen Verfahren erzielt werden können, ist es ebenso von Vorteil, dass Lösungsmittelgemische aus dipolar aprotischem Lösungsmittel und Substanzen wie z.B. Alkoholen oder Wasser gut recyclebar sind und dadurch nur wenig Lösungsmittel für die Herstellung der Ester notwendig ist. Ein weiterer Vorteil des erfindungsgemäßen Verfahrens ist, dass auf den Einsatz von geruchlich und toxikologisch problematischen Aminen verzichtet werden kann.

Vorzugsweise wird als Lösungsmittel 2-Methylpyrrolidon (NMP) verwendet.

Das erfindungsgemäße Verfahren zur Veresterung von Sacchariden umfasst die Veresterung von Monosacchariden sowie von Oligo- und Polysacchariden wie z.B. von Disacchariden.

Vorzugsweise werden nach dem erfindungsgemäßen Verfahren Oligo- und Polysaccharide einschließlich Disacchariden verestert.

Unter den Disacchariden werden vorzugsweise Rohrzucker, Milchzucker Trehalose, Lactose, Maltose, Gentiobiose, Melibiose und Cellobiose in dem erfindungsgemäßen Verfahren verwendet.

Neben den Disacchariden werden unter den Oligo- und Polysacchariden vorzugsweise Cellotriose, Cellotetrose, Raffinose, Acarbose, aber auch Stärke und deren Bestandteile Amylose, Amylopektin, sowie Dextrine, Dextrane, Xanthane oder auch Cellulose in dem erfindungsgemäßen Verfahren verwendet.

In einer bevorzugten Ausführungsform der Erfindung werden Saccharide ausgewählt aus Dextrin und Cellobiose in dem Verfahren verwendet, d.h. es werden Verbindungen ausgewählt aus Dextrinestern und Cellobioseestern hergestellt. Die Dextrine haben vorzugsweise einen Polymerisationsgrad von 3 bis 200, besonders bevorzugt von 5 bis 100 und insbesondere bevorzugt von 10 bis 50.

Zur Veresterung der Mono-, Oligo- und Polysaccharide werden in einer bevorzugten Ausführungsform der Erfindung aktivierte gesättigte oder ungesättigte aliphatische, cyclisch aliphatische oder aromatische Carbonsäuren mit 2 bis 30 Kohlenstoffatomen eingesetzt.

Als den aktivierten Carbonsäuren zugrunde liegende Säuren seien beispielsweise genannt Caprylsäure, Caprinsäure, Pelargonsäure, Laurylsäure, Laurinsäure, Myristinsäure, Myristylsäure, Palmitinsäure, Palmitylsäure, Stearinsäure, Arachinsäure, Behensäure, Ölsäure, Erucasäure, Gadoleinsäure, Linocerylsäure, Fischölsäure und Sojaölfettsäure sowie deren Derivate.

Als weitere den aktivierten Carbonsäuren zugrunde liegende Säuren seien beispielsweise genannt kurzkettige Säuren, z.B. Essigsäure, Propionsäure und Buttersäure sowie deren Derivate.

Als weitere den aktivierten Carbonsäuren zugrunde liegende Säuren seien beispielsweise gennant verzweigte, gesättigte Carbonsäuren, z.B. Isobuttersäure, Isovaleriansäure, 2-Ethylbuttersäure, Ethylmethylessigsäure, Isoheptylsäure, 2-Ethylhexylsäure, Isononansäure, Isodecansäure, Isotridecansäure, Isomyristylsäure, Isopalmitylsäure, Isostearinsäure, Isoarachinsäure und Isohexacosansäure sowie deren Derivate.

Als weitere den aktivierten Carbonsäuren zugrunde liegende Säuren seien beispielsweise genannt ungesättigte Carbonsäuren, z.B. cis-4-Decensäure, 9-Decensäure, cis-4-Dodecensäure, cis-4-Tetradecensäure, cis-5-Tetradecensäure, cis-9-Tetradecensäure, cis-6-Hexadecensäure, cis-9-Hexadecensäure, cis-9-Octadecensäure, trans-9-Octadecensäure, cis-11-Octadecensäure, cis-11-Eicosensäure, cis-17-Hexacosensäure und cis-21-Tricontensäure, aber auch Sorbinsäure, Linolsäure, Linolensäure, .gamma.-Linolensäure, Stearidonsäure, Arachidonsäure und Clupanodonsäure sowie deren Derivate.

Als weitere den aktivierten Carbonsäuren zugrunde liegende Säuren seien beispielsweise genannt aromatische Carbonsäuren, z.B. Benzoesäure, Anthranilsäure, Salicylsäure, Phthalsäure, Terephthalsäure, Isophthalsäure sowie deren Derivate und araliphatische Monocarbonsäuren, z.B. Zimtsäure und Mandelsäure sowie deren Derivate.

Besonders bevorzugte Säuren, die den im erfindungsgemäßen Verfahren verwendeten aktivierten Carbonsäuren zugrunde liegen, sind geradkettige oder verzweigte, gesättigte oder ungesättigte Fettsäuren mit 8 bis 22 Kohlenstoffatomen.

In einer weiteren bevorzugten Ausführungsform der Erfindung werden Saccharide, vorzugsweise ausgewählt aus Disacchariden, darunter bevorzugt Cellobiose, sowie Oligo- und Polysacchariden mit Polymerisationsgraden von 3 bis 200, darunter bevorzugt Dextrine, mit einer Kombination von mehreren aktivierten Carbonsäuren verestert.

Als hierdurch erhältliche Reaktionsprodukte seien beispielhaft folgende Dextrinestergemische genannt: Dextrinester der Capryl-/Isobuttersäure, Dextrinester der Capryl-/2-Ethylhexylsäure, Dextrinester der Capryl-/Isoarachinsäure, Dextrinester der Capryl-/Linolsäure, Dextrinester der Capryl-/Essigsäure, Dextrinester der Capryl-/Isopalmityl-/Buttersäure, Dextrinester der Capryl-/Palmityl-/Ölsäure, Dextrinester der Capryl-/Öl-/Essigsäure, Dextrinester der Lauryl-/Ethylmethylessigsäure, Dextrinester der Lauryl-/2-Ethylhexylsäure, Dextrinester der Lauryl-/Caprinsäure, Dextrinester der Lauryl-/Linolen-/Propionsäure, Dextrinester der Lauryl-/Behen-/Isoheptansäure, Dextrinester der Myristyl-/Isostearinsäure, Dextrinester der Myristyl-/Isohexansäure, Dextrinester der Myristyl-/Arachidonsäure, Dextrinester der Palmitin-/2-Ethylhexansäure, Dextrinester der Palmitin-/Isostearinsäure, Dextrinester der Palmitin-/Ölsäure, Dextrinester der Palmitin-/Isovalerian-/Isostearinsäure, Dextrinester der Palmitin-/Isononan-/Capronsäure, Dextrinester der Palmitin-/Stearin/2-Ethylhexansäure, Dextrinester der Palmitin-/Stearin-/Capronsäure, Dextrinester der Stearin-/Isopalmitinsäure, Dextrinester der Stearin-/Ölsäure, Dextrinester der Behen-/2-Ethylbuttersäure und Dextrinester der Behen-/Capron-Naleriansäure.

Sämtliche soeben genannten Dextrinestergemische können vorteilhafterweise z.B. auch auf Basis von Disacchariden, insbesondere von Cellobiose, hergestellt werden.

In einer weiteren bevorzugten Ausführungsform der Erfindung werden Cellobioseester gemäß Formel I hergestellt 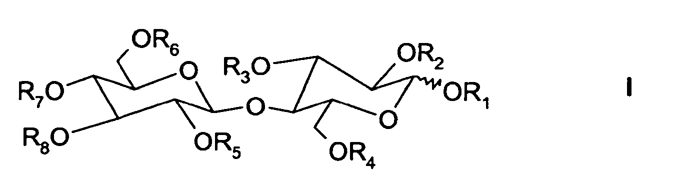 worin die Reste R₁ bis R₈ jeweils unabhängig voneinander für H oder für einen Acylrest stehen und die an die CO-Gruppe gebundenen Gruppen der Acylreste ausgewählt sind aus linearen oder verzweigten gesättigten und ungesättigten Kohlenwasserstoffgruppen mit 1 bis 29, bevorzugt 7 bis 21 Kohlenstoffatomen oder aus cyclischen oder aromatischen Kohlenwasserstoffgruppen mit 4 bis 29 Kohlenwasserstoffatomen mit der Maßgabe, dass die Anzahl der Acylgruppen größer als 0 ist.

In einer weiteren bevorzugten Ausführungsform der Erfindung werden Oligo- oder Polysaccharide gemäss Formel II hergestellt 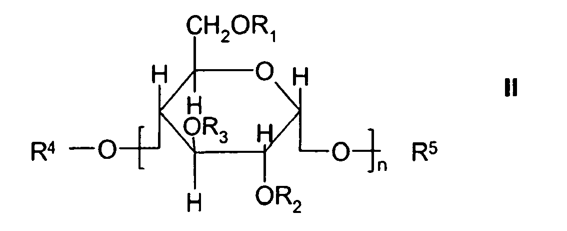 worin die Reste R₁ bis R₅ jeweils unabhängig voneinander für H oder für einen Acylrest stehen und die an die CO-Gruppe gebundenen Gruppen der Acylreste ausgewählt sind aus linearen oder verzweigten gesättigten und ungesättigten Kohlenwasserstoffgruppen mit 1 bis 29, bevorzugt 7 bis 21 Kohlenstoffatomen oder aus cyclischen oder aromatischen Kohlenwasserstoffgruppen mit 4 bis 29 Kohlenstoffatomen und der Polymerisationsgrad n vorzugsweise 3 bis 200, besonders bevorzugt 5 bis 100 und außerordentlich bevorzugt 10 bis 50 beträgt mit der Maßgabe, dass die Anzahl der Acylgruppen größer als 0 ist.
In einer besonders bevorzugten Ausführungsform der Erfindung werden aktivierte Carbonsäuren, deren zugrunde liegende Säure Palmitinsäure ist, in dem erfindungsgemäßen Verfahren verwendet. Außerordentlich bevorzugt werden nach dem erfindungsgemäßen Verfahren Substanzen ausgewählt aus Dextrinpalmitat und Cellobiosepalmitat hergestellt. Hierbei haben die Dextrine vorzugsweise einen Polymerisationsgrad von 3 bis 200, besonders bevorzugt von 5 bis 100 und insbesondere bevorzugt von 10 bis 50.

Der Veresterungsgrad pro Zuckereinheit ist bei Disacchariden vorzugsweise von 0,1 bis 4 und besonders bevorzugt von 2 bis 4.

Bei Oligo- und Polysacchariden mit Polymerisationsgraden von 3 bis 200 ist der Veresterungsgrad pro Zuckereinheit vorzugsweise mindestens 0,1, d.h. von 0,1 bis 3,67 bei Sacchariden mit n = 3, von 0,1 bis 3,5 bei Sacchariden mit n = 4, etc. Bei Oligo- und Polysacchariden mit Polymerisationsgraden von 3 bis 200 ist der Veresterungsgrad pro Zuckereinheit besonders bevorzugt von 0,1 bis 3 und außerordentlich bevorzugt von 2 bis 3.

In dem erfindungsgemäßen Verfahren beträgt das molare Verhältnis von aktivierter Carbonsäure zu Zuckereinheit bei der Verwendung von Disacchariden vorzugsweise von 0,1 : 1 bis 4 : 1 und besonders bevorzugt von 2 : 1 bis 4 : 1.

In dem erfindungsgemäßen Verfahren ist das molare Verhältnis von aktivierter Carbonsäure zu Zuckereinheit bei der Verwendung von Oligo- und Polysacchariden mit Polymerisationsgraden von 3 bis 200 vorzugsweise mindestens 0,1 : 1. In dem erfindungsgemäßen Verfahren ist das molare Verhältnis von aktivierter Carbonsäure zu Zuckereinheit bei der Verwendung von Oligo- und Polysacchariden mit Polymerisationsgraden von 3 bis 200 besonders bevorzugt von 0,1 : 1 bis 3 : 1 und außerordentlich bevorzugt von 2 : 1 bis 3 : 1.

In einer weiteren bevorzugten Ausführungsform der Erfindung wird Cellobioseoctanonanoat hergestellt.

Das erfindungsgemäße Verfahren wird vorzugsweise bei einer Temperatur von 45 bis 80°C, besonders bevorzugt bei einer Temperatur von 65 bis 75°C, durchgeführt.

In einer weiteren bevorzugten Ausführungsform wird das Verfahren derart ausgeführt, dass das Lösungsmittel bzw. Lösungsmittelgemisch recycliert wird.

Die nachfolgenden Beispiele sollen den Gegenstand der Erfindung näher erläutern, ohne ihn jedoch darauf einzuschränken.

Die Entwässerung der Zuckerkomponente kann sowohl mit und ohne Schleppmittel (Petrolether) erfolgen, wie in den Beispielen dargestellt. Auch für die Neutralisation der Reaktionsmischung kann sowohl eine wässrige Base, als auch eine alkoholische Base eingesetzt werden. Dies soll in den Beispielen demonstriert werden.

### Beispiel 1 - Herstellung von Cellobiosepalmitat

In einen Rührkolben mit Vakuumwechselvorstoß werden 12,5 g wasserhaltige Cellobiose und 120 g 2-Methylpyrrolidon (NMP) vorgelegt und bei maximal 110°C und 50 mbar 20 % des eingesetzten NMP wieder abdestilliert. Anschließend wird der Ansatz auf 70°C abgekühlt und innerhalb von 60 Minuten bei 70 - 75°C 1 mol Palmitinsäurechlorid pro Hydroxylgruppe der Cellobiose zudosiert. Nach 4 Stunden Nachreaktion bei 70 - 75°C wird der Ansatz unter gleichzeitiger Dosierung von 23 g NaOH (50 gew.-%ig) in 200 g vorgelegtes Isopropanol getropft (Dauer: 45 - 60 Minuten). Der pH-Wert liegt zwischen 2 und 4. Am Ende dieser Fällungsreaktion wird dieses isopropanolische Gemisch mit NaOH (50 gew.-%ig) auf pH 10 (10 % tq wässrig) eingestellt. Anschließend wird der Niederschlag abgesaugt und mit VE-Wasser (eingestellt auf pH 10) und reinem VE-Wasser jeweils 30 Minuten bei 40 - 45°C gewaschen. Der gewaschene Niederschlag wird über Nacht bei 50°C im Vakuum (20 - 50 mbar) getrocknet. Die Ausbeute an feinkristallinem Produkt beträgt 70 % (Schmelzpunkt ca. 120°C).

### Beispiel 2 - Herstellung von Dextrinpalmitat

In einen Rührkolben mit Wasserabscheider werden wasserhaltiges Dextrin (17,8 g) und Petrolether (Siedebereich 70 - 90°C) vorgelegt und 4 Stunden bei 74 - 78°C Wasser ausgekreist. Anschließend werden ca. 75 % des eingesetzten Petrolethers wieder abdestilliert. Es werden 100 g 2-Methylpyrrolidon (NMP) zugegeben und bis zu einer Sumpftemperatur von 130°C der restliche Petrolether abdestilliert. Danach wird der Ansatz auf 70°C abgekühlt und innerhalb von 60 Minuten bei 70 - 75°C Palmitinsäurechlorid (82,5 g) zudosiert. Nach 4 Stunden Nachreaktion bei 70 - 75°C wird der Ansatz innerhalb von 45 - 60 Minuten auf 150 g Isopropanol dosiert, bei gleichzeitiger Dosierung von isopropanolischer KOH (15 gew.-%ig) bei pH 2 - 4. Der dabei entstehende Niederschlag wird anschließend mit isopropanolischer KOH auf pH 10 (10 % tq wässrig) eingestellt und abgesaugt. Der Niederschlag wird mit VE-Wasser (eingestellt auf pH 10) und reinem VE-Wasser 30 Minuten bei 40 - 45°C gewaschen. Danach wird der gewaschene Niederschlag über Nacht bei 50°C und 20 - 50 mbar getrocknet. Die Ausbeute an Dextrinpalmitat beträgt 79,5 %.

Vergleichsbeispiel - Herstellung von Dextrinpalmitat in Gegenwart von Pyridin 16,2 g getrocknetes Dextrin und 94,9 g wasserfreies Pyridin werden in einem 500 ml-Rührkolben vorgelegt. Anschließend wird unter Rühren langsam 82,5 g Palmitinsäurechlorid zugetropft. Es tritt eine leichte Erwärmung auf 40 - 50°C ein. Die Mischung wird 4 Stunden bei 60°C gerührt. Danach wird der Ansatz auf 350 g Wasser gegossen und mit 85 g konz. Salzsäure auf pH 1,6 - 2,0 eingestellt. Es wird einige Minuten nachgerührt, anschließend abgesaugt und das feuchte Produkt in 300 g Petrolether (Siedebereich 60 - 70°C) gelöst. Nun wird der Ansatz mit 300 g 1 %iger Salzsäure versetzt und 0,5 Stunden bei 40°C gerührt. Danach wird diese Mischung in einen 2-Liter-Scheidetrichter überführt, die organische Phase von der wässrigen Phase getrennt und die organische Phase am Rotationsverdampfer bei leichtem Vakuum bei 30 - 40°C einrotiert. Anschließend wird der feste Rückstand in 300 g Essigsäureethylester (wasserfrei) aufgenommen und umkristallisiert. Die Umkristallisation wird gegebenenfalls wiederholt.

Gegenüberstellung der Produkte und Ausbeuten aus der Pyridin-Variante und der 2-Methylpyrrolidon (NMP)-Variante:

| | Pyridin-Variante (Vergleichsbeispiel) | NMP-Variante (Beispiel 2) |
|---|---|---|
| Aussehen bei Raumtemperatur | hellgelbes Pulver | weiß, pulvrig bis körnig |
| Ausbeute | 37,0 % | 79,5 % |
| Säurezahl | 3,4 mgKOH/g | 0,92 mgKOH/g |
| Chloridgehalt | nicht bestimmt | 0,10 % |
| Schmelzpunkt | ca. 99°C | ca. 97°C |

## Patentansprüche

1. Verfahren zur Herstellung von Saccharidestern durch Umsetzung von Sacchariden mit aktivierten Carbonsäuren ausgewählt aus Carbonsäurechloriden in einem dipolar aprotischen Lösungsmittel ausgewählt aus der Gruppe der Lactame und in Abwesenheit einer basischen Aminverbindung.

2. Verfahren nach Anspruch 1, **dadurch gekennzeichnet, dass** das Lactam 2-Methylpyrrolidon ist.

3. Verfahren nach Anspruch 1 oder 2, **dadurch gekennzeichnet, dass** die Saccharide ausgewählt sind aus Dextrin und Cellobiose.

4. Verfahren nach einem oder mehreren der Ansprüche 1 bis 3, **dadurch gekennzeichnet, dass** die den aktivierten Carbonsäuren zugrunde liegende Säure Palmitinsäure ist.

5. Verfahren nach einem oder mehreren der Ansprüche 1 bis 4, **dadurch gekennzeichnet, dass** das molare Verhältnis von aktivierter Carbonsäure zu Zuckereinheit bei der Verwendung von Disacchariden von 0,1 : 1 bis 4 : 1 beträgt.

6. Verfahren nach einem oder mehreren der Ansprüche 1 bis 4, **dadurch gekennzeichnet, dass** das molare Verhältnis von aktivierter Carbonsäure zu Zuckereinheit bei der Verwendung von Oligo- oder Polysacchariden mit Polymerisationsgraden von 3 bis 200 mindestens 0,1 : 1 ist.

7. Verfahren nach einem oder mehreren der Ansprüche 1 bis 6, **dadurch gekennzeichnet, dass** es bei einer Temperatur von 45 bis 80°C durchgeführt wird.

8. Verfahren nach einem oder mehreren der Ansprüche 1 bis 7, **dadurch gekennzeichnet, dass** das Lösungsmittel recycliert wird.

9. Verfahren nach einem oder mehreren der Ansprüche 1 bis 3, 5, 7 und 8, **dadurch gekennzeichnet, dass** Cellobioseoctanonanoat hergestellt wird.

## Claims

1. A process for the preparation of saccharide esters by reacting saccharides with activated carboxylic acids chosen from carbonyl chlorides in a dipolar-aprotic solvent chosen from the group of lactams and in the absence of a basic amine compound.

2. The process as claimed in claim 1, wherein the lactam is 2-methylpyrrolidone.

3. The process as claimed in claim 1 or 2, wherein the saccharides are chosen from dextrin and cellobiose.

4. The process as claimed in one or more of claims 1 to 3, wherein the acid on which the activated carboxylic acids are based is palmitic acid.

5. The process as claimed in one or more of claims 1 to 4, wherein the molar ratio of activated carboxylic acid to sugar unit in the case of the use of disaccharides is from 0.1:1 to 4:1.

6. The process as claimed in one or more of claims 1 to 4, wherein the molar ratio of activated carboxylic acid to sugar unit in the case of the use of oligo- or polysaccharides with degrees of polymerization of from 3 to 200 is at least 0.1:1.

7. The process as claimed in one or more of claims 1 to 6, which is carried out at a temperature of from 45 to 80°C.

8. The process as claimed in one or more of claims 1 to 7, wherein the solvent is recycled.

9. The process as claimed in one or more of claims 1 to 3, 5, 7 and 8 wherein cellobiose octanonanoate is prepared.

## Revendications

1. Procédé de fabrication d'esters de saccharides par la transformation de saccharides avec des acides carboxyliques activés choisis parmi des chlorures d'acides carboxyliques dans un solvant aprotique dipolaire choisi dans le groupe des lactames et en l'absence d'un composé aminé basique.

2. Procédé selon la revendication 1, **caractérisé en ce que** le lactame est du 2-méthyle-pyrrolidone.

3. Procédé selon la revendication 1 ou 2, **caractérisé en ce que** les saccharides sont choisis parmi la dextrine et la cellobiose.

4. Procédé selon une ou plusieurs des revendications 1 à 3, **caractérisé en ce que** l'acide à la base des acides carboxyliques activés est l'acide palmitique.

5. Procédé selon une ou plusieurs des revendications 1 à 4, **caractérisé en ce que** le rapport molaire de l'acide carboxylique activé sur l'équivalent de sucre lors de l'utilisation de disaccharides est de 0,1:1 à 4:1.

6. Procédé selon une ou plusieurs des revendications 1 à 4, **caractérisé en ce que** le rapport molaire de l'acide carboxylique activé sur l'équivalent de sucre lors de l'utilisation d'oligosaccharides ou de polysaccharides avec des degrés de polymérisation de 3 à 200 est au moins de 0,1:1.

7. Procédé selon une ou plusieurs des revendications 1 à 6, **caractérisé en ce qu'**il est réalisé à une température de 45 à 80°C.

8. Procédé selon une ou plusieurs des revendications 1 à 7, **caractérisé en ce que** le solvant est recyclé.

9. Procédé selon une ou plusieurs des revendications 1 à 3, 5, 7 et 8, **caractérisé en ce qu'**il est fabriqué de l'octanonanoate de cellobiose.
